(19) Europäisches Patentamt
European Patent Office
Office européen des brevets

(11) Publication number: 0 199 096 B1

# (12) EUROPEAN PATENT SPECIFICATION

(45) Date of publication of the patent specification: 20.12.89

(51) Int. Cl.⁴: **C07D 401/04**, C07D 403/04, A61K 31/495, A61K 31/55

(21) Application number: 86103790.1

(22) Date of filing: 20.03.86

(54) **Nitro substituted heteroaryl compounds, process for their preparation and pharmaceutical compositions containing them.**

(30) Priority: 27.03.85 US 716884

(43) Date of publication of application: 29.10.86 Bulletin 86/44

(45) Publication of the grant of the patent: 20.12.89 Bulletin 89/51

(84) Designated Contracting States: CH DE FR GB IT LI NL

(56) References cited:
EP-A- 0 019 578
US-A- 3 830 786
US-A- 4 163 849

CHEMICAL ABSTRACTS, vol. 85, no. 1, July 5, 1976, Columbus, Ohio, USA CIGNARELLA, G.; PIRISINO, G.; LORIGA, M. "2,6-Dialkylpiperazines. VII.Synthesis of N4-(2'-pyridyl)-2,6-dimethylpiperazine and separation of its geometrical isomers." page 451, column 1, abstract no. 5590g muscle" Seite 34, Spalte 2, Zusammenfassung-Nr. 400j 000

(73) Proprietor: MERCK & CO. INC., 126, East Lincoln Avenue P.O. Box 2000, Rahway New Jersey 07065-0900(US)

(72) Inventor: Engelhardt, Edward L., 904 Plymouth Road, Gwynedd Valley, PA 19434(US)
Inventor: Saari, Walfred S., 1740 Wagon Wheel Lane, Lansdale, PA 19446(US)

(74) Representative: Blum, Rudolf Emil Ernst et al, c/o E. Blum & Co Patentanwälte Vorderberg 11, CH-8044 Zürich(CH)

EP 0 199 096 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

**Description**

BACKGROUND OF THE INVENTION

This invention relates to nitrophyrazinyl- and nitropyridinyl-substituted piperazin-3-one and hexahydro-1H-1,4-diazepin-5-one compounds used as sensitizers of tumor cells to therapeutic radiation. It also relates to the process of preparing such compounds hy reaction of substituted piperazin-3-one and hexahydro-1H-1,4-diazepin-5-one compounds with certain specific chloronitropyrazines and chloronitropyrines defined hereinbelow.

DESCRIPTION OF THE PRIOR ART

At the present time certain compounds, which are not structurally related to the inventive nitro substituted heterocyclic compounds are in experimental clinical use as radiation sensitizers. However, these compounds – for example metronidazole and misonidazole – suffer from the drawback that they also cause neurotoxicity which limits their usefulness.

In the U.S. patent 4 163 849 there are described substituted piperazinones, in which to one nitrogen atom of said heterocycle is bonded a 2-pyrazinyl radical, which is chlorosubstituted in its 6-position, and to the other nitrogen atom of said heterocycle an alkyl group having 1–4 carbon atoms. Said compounds are anorexigenic, antidepressant, analgesic and hypnotic agents.

In the European patent publication 0 019 578 there are described several heterocyclic compounds, which can be also corresponding hexahydro-1H-1,4-diazepin-5-ones, in which the nitrogen atom in the 1-position and also the nitrogen atom in the 4-position is substituted with a 4-piperidine group. Said compounds are used as light stabilizers, heat stabilizers and oxidation stabilizers in synthetic polymer materials.

2,6 dimethylpiperazine-3-ones which have an unsubstituted 2-pyridyl residue bonded to the nitrogen atom in the 4-position are furthermore disclosed in "Chemical Abstracts", volume 85, no. 1, page 451, column 1, abstract no. 5590g and in the U.S. patent 3 830 786 there are described several heterocyclic compounds, including hexahydro-1,4-diazepin-3-ones, which can be used for the preparation of film-forming and fibre-forming polyamides.

DESCRIPTION OF THE INVENTION

It was the aim of the present invention to provide compounds which are effective radiation sensitizers which do not have the undesired side-effects of radiation sensitizers until now in clinical use.

One subject of the present invention are nitro substituted heterocyclic compounds of formula I

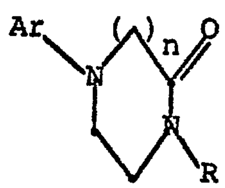

I

wherein

n is the integer 1 or 2,

R is hydrogen or a hydroxyethyl group and

Ar is a heteroaryl group selected from 3-nitro-2-pyrazinyl and 3-nitro-4-pyridyl and salts of the compound of formula I.

A further subject of the present invention is a process for the preparation of the compounds of formula I which is characterized in that the lactam of formula II

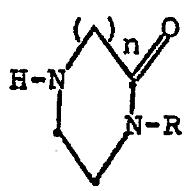

II

wherein

n and R have the same meaning as in formula I, is reacted either with a 3-nitropyrazine having a leaving group in the 2-position or with a 3-nitropyridine having a leaving group in the 4-position, in the presence of an organic solvent and an organic or inorganic base or in the presence of a basic organic solvent and wherein the compounds of formula I are isolated in the free form or as salts thereof.

The reaction is carried out in a suitable solvent such as a lower alcohol, a polar solvent such as

dimethylformamide, dimethylsulfoxide, tetramethylurea, or others such as tetrahydrofuran, glyme, diglyme and pyridine.

A preferred solvent for this reaction is isopropyl alcohol.

The reaction mixture should also contain sufficient base to neutralize the hydrochloric acid formed during the reaction. This base may be in the form of an organic amine such as pyridine or triethylamine or inorganic base such as an alkali metal carbonate, bicarbonate or hydroxide with organic bases being preferred.

The temperature of the reaction is not critical and it is desirably maintained between 0 and 100°c and preferably between 0 and 25°C for a period of from 1–25 hours.

A further object of the present invention is a pharmaceutical composition for enhancing the therapeutic effect of a radiation treatment of malignant diseases, which pharmaceutical composition is characterized in that it contains as pharmaceutically effective ingredient a compound of formula I according to claim 1 or a pharmaceutically acceptable salt thereof.

The inventive pharmaceutical composition can be administered orally or intravenously. The dose in which it is employed depends on the radiation protocol for each individual patient. In protocols where the radiation dose is divided into a large number of fractions, the composition can be administered at intervals in the schedule and not necessarily with each radiation treatment. It should be noted that the pharmaceutical compositions of the present invention are not intended for chronic administration. In general, they are administered from 10 minutes to 5 hours prior to the radiation treatment in such an amount that there is administered a dosage of 0.25 to about 4.0 grams of the aotive ingredient per square meter of body surface.

The dosage range given is the effective dosage range and the decision as to the exact dosage used must be made by the administering physician based on his judgement of the patient's general physical condition. In determining the dose for the individual patient, the physician may begin with an initial dose of 0.25 g/square meter of body surface to determine how well the drug is tolerated and increase the dosage with each suceeding radiation treatment, observing the patient carefully for any drug side effect. The composition to be administered is an effective amount of the active compound and a pharmaceutical carrier for said active compound.

The dosage form for intravenous administration is a sterile isotonic solution. Oral dosage forms such as tablets, capsules, or elixirs are preferred.

Capsules or tablets containing 25, 50, 100 or 500 mg of drug/capsule or tablet are satisfactory for use in the method of treatment of our invention.

The following examples are intended to illustrate but do not limit the process of preparation, product, compositions, or method of treatment aspects of the invention. Temperatures are in degrees Celsius unless otherwise indicated throughout the application.

EXAMPLE 1

1-(3-Nitro-2-pyrazinyl)piperazin-3-one

A solution of 2-chloro-3-nitropyrazine (9.1. g, 57 mmol) in chloroform (100 ml) was added dropwise over 45 minutes ot a cooled solution of piperazine-2-one (5.71 g, 57 mmol) and triethylamine (8.0 ml, 57 mmol) in isopropanol (120 ml). After addition was complete, the reaction mixture was allowed to warm to 20-25° and was stirred at this temperature for 20 hours. The precipitate was removed by filtration and recrystallized from methanol-ethyl acetate-hexane to give 6.96 g (54.7%) of product, m.p. 178-179°.

Anal. Calcd. for $C_8H_9N_5O_3$: C, 43.05; H, 4.06; N, 31.38.

Found: C, 42.88; H, 4.10; N, 31.19.

EXAMPLE 2

1-(3-Nitro-2-pyrazinyl)hexahydro-1H-1,4-diazepin-5-one

To a solution of hexahydro-1H-1,4-diazepin-5-one (1.14 g, 10 mmol) and triethylamine (1.01 g, 10 mmol) in isopropanol (30 ml) cooled in an ice bath, was added over 30 minutes a solution of 2-chloro-3-nitropyrazine (1.59 g, 10 mmol) in chloroform (20 ml). After addition was complete, the reaction mixture was allowed to warm to 20-25° and was stirred at this temperature for 20 hours. Solvents were removed under reduced pressure and the residue was partitioned between a saturated aqueous solution of sodium chloride and ethyl acetate. The ethyl acetate extract was dried over anhydrous sodium sulfate, filtered and concentrated to an oil. Flash chromatography over silica gel and elution with 2% methanol -98% chloroform gave pure 1-(3-nitro-2-pyrazinyl)hexahydro-1H-1,4-diazepin-5-one.

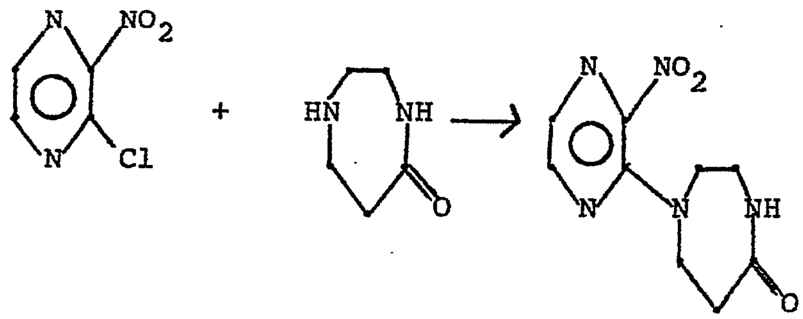

EXAMPLE 3

1-(3-Nitro-2-pyrazinyl)-4-(2-hydroxyethyl)piperazin-3-one

Step A: 1-Carbobenzoxy-4-(2-hydroxyethyl)piperazine-3-one

To a solution of 1-carbobenzoxypiperazin-3-one (234 mg, 1.0 mmol) in dimethylformamide (10 ml) under nitrogen was added in one portion, 50% sodium hydride (48 mg, 1 mmol). After stirring at 20-25° for 30 minutes until all of the sodium hydride had reacted, a solution of 2-(2-bromoethoxy)-tetrahydropyran (209 mg, 1 mmol) in dimethylformamide (2 ml) was added and the reaction mixture stirred at 20-25° for 20 hours. Solvent was removed at 40-45° and 0.1 mm and the residue chromatographed over silica gel. Elution with 2% isopropanol -98% methylene chloride gave the pure protected alcohol as an oil.

The tetrahydropyranyl blocking group was removed by heating a solution of the protected alcohol (200 mg, 0.55 mmol) in a mixture of acetic acid (8 ml), tetrahydrofuran (4 ml) and water (2 ml) at 50°C for 4 hours. After removing solvents under reduced pressure, the residue was partitioned between saturated sodium bicarbonate solution and ethyl acetate. The ethyl acetate extract was dried over anhydrous sodium sulfate, filtered and concentrated. Chromatography of the residue over silica gel and elution with 5% methanol-95% chloroform gave pure 1-carbobenzoxy-4-(2-hydroxyethyl)piperazine-3-one.

Step B: 4-(2-hydroxyethyl)piperazin-3-one

A solution of 1-carbobenzoxy-4-(2-hydroxyethyl)piperazine-3-one (1.0 g, 35.9 mmol) in absolute ethanol (75 ml) was hydrogenated over a 10% palladium on carbon catalyst (1.0 g) at 20–25° and an initial pressure of 2,81 kg/cm$^2$ (2,76 · $10^5$ Pa or 40 psi) for 6 hours. Catalyst was removed by filtration through diatomaceous earth and the filtrate concentrated under reduced pressure to give 4-(2-hydroxyethyl)piperazine-3-one.

Step C: 2-(3-Nitro-2-pyrazinyl)-4-(2-hydroxyethyl) piperazin-3-one

A solution of 2-chloro-3-nitropyrazine (1.59 g, 10 mmol) in chloroform (20 ml) was added over 30 minutes to an icebath cooled solution of 4-(2-hydroxyethyl)piperazin-3-one (1,44 g, 10 mmol) and triethylamine (1.01 g, 10 mmol) in isopropanol (30 ml). After addition was complete, the reaction mixture was allowed to warm to 20–25° and was stirred at this temperature for 20 hours. Solvents were removed under reduced pressure and the residue was partitioned between a saturated aqueous solution of sodium chloride and ethyl acetate. The ethyl acetate extract was dried over anhydrous sodium sulfate, filtered and concentrated to an oil. Flasch chromatography over silica gel and elution with 5% methanol-95% chloroform gave 0.90 g (48.6%) of 1-(3-nitro-2-pyrazinyl)-4-(2-hydroxyethyl)piperazin-3-one. An analytical sample, m.p. 117-120°, was obtained upon recrystallization from ethylacetatehexane.

EXAMPLE 4

1-(3-Nitro-4-pyridyl)piperazin-3-one

A solution of 4-chloro-3-nitropyridine (434 mg, 2.74 mmol) in 2 ml of dimethylformamide was added dropwise to a stirred suspension of 548 mg (5.47 mmol) of piperazin-2-one in 2 ml of dimethylformamide at room temperature (approximately 25°) over a period of 30 minutes. A voluminous bright yellow solid began to separate when about half of the solution had been added. After stirring at room temperature for 20 hours, the dimethylformamide was removed by passing a slow stream of nitrogen over the surface of the reaction mixture while heating in a bath at 80° for 18 hours. The yellow crystalline residue, with a small amount of dark brown material on the surface, was recrystallized from 5 ml of water to give 536 mg of product that sintered at 201° and decomposed at 204°. A second recrystallization from water, 27 ml, gave 453 mg of bright yellow needles, dec. 212°, with effervescence.

## Claims

1. Nitro substituted heterocyclic compound of formula I

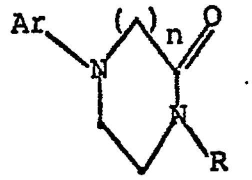

I

wherein
n is the integer 1 or 2,
R is hydrogen or a hydroxyethyl group and
Ar is a heteroaryl group selected from 3-nitro-2-pyrazinyl and 3-nitro-4-pyridyl and
salts of the compounds of formula I.

2. A compound according to claim 1 which is 1-(3-nitro-2-pyrazinyl)piperazin-3-one.

3. A compound according to claim 1 which is 1-(3-nitro-2-pyrazinyl)hexahydro-1H-1,4-diazepin-5-one.

4. A compound according to claim 1 which is 1-(3-nitro-2-pyrazinyl)-4-(2-hydroxyethyl)piperazin-3-one.

5. A compound according to claim 1 which is 1-(3-nitro-4-pyridyl)piperazin-3-one.

6. A process for the preparation of the compounds of formula I

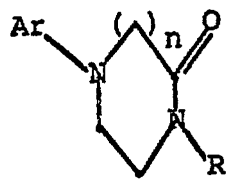

I

according to claim 1, wherein
n, R and Ar have the same meaning as defined in claim 2 or salts of the compounds of formula I, characterized in that the lactam of the formula II

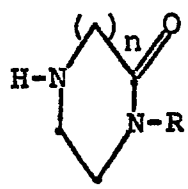

II

wherein
n and R have the same meaning as in formula I, is reacted either with a 3-nitropyrazine having a leaving group in the 2-position or with a 3-nitropyridine having a leaving group in the 4-position, in the presence of an organic solvent and an organic or inorganic base or in the presence of a basic organic solvent and wherein the compounds of formula I are isolated in the free form or as salts thereof.

7. Process according to claim 6, characterized in that the leaving group of the 3-nitropyrazine-starting material respectively 3-nitropyridine-starting material is a chlorine atom and that the reaction is performed in the presence of a lower alcohol, preferably isopropyl alcohol or a polar organic solvent.

8. Pharmaceutical composition for enhancing the therapeutic effect of a radiation treatment of malignant diseases, characterized in that the pharmaceutical composition contains as pharmaceutically effective ingredient a compound of formula I according to claim 2 or a pharmaceutically acceptable salt thereof.

9. Pharmaceutical composition according to claim 8, characterized in that it contains as pharmaceutically active ingredient a compound according to one of the claims 2, 3, 4 or 5.

10. Pharmaceutical composition according to claim 8 or 9, characterized in that it furthermore contains a pharmaceutically acceptable carrier.

## Patentansprüche

1. Nitro-substituierte, heterocyclische Verbindung der Formel I

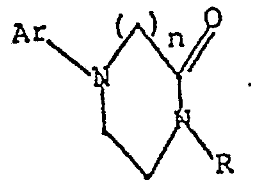

I

worin n die Zahl 1 oder 2 ist,

R Wasserstoff oder eine Hydroxyethylgruppe ist

und

Ar eine aus 3-Nitro-2-pyrazinyl und 3-Nitro-4-pyridyl ausgewählte Heteroarylgruppe ist,

sowie Salze der Verbindungen der Formel I.

2. Verbindung nach Anspruch 1, welche 1-(3-Nitro-2-pyrazinyl)piperazin-3-on ist.

3. Verbindung nach Anspruch 1, welche 1-(3-Nitro-3-pyrazinyl)hexahydro-1H-1,4-diazepin-5-on ist.

4. Verbindung nach Anspruch 1, welche 1-(3-Nitro-2-pyrazinyl)-4-(2-hydroxyethyl)piperazin-3-on ist.

5. Verbindung nach Anspruch 1, welche 1-(3-Nitro-4-pyridyl)piperazin-3-on ist.

6. Verfahren zur Herstellung der Verbindungen der Formel I

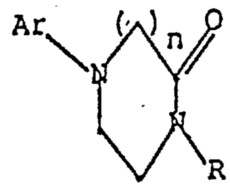

I

nach Anspruch 1, worin n, R und Ar die gleiche Bedeutung haben, wie in Anspruch 1 definiert, oder Salze der Verbindungen der Formel I, dadurch gekennzeichnet, daß das Lactam der Formel II

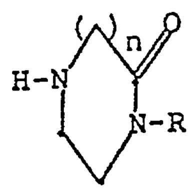

II

worin n und R die gleiche Bedeutung haben wie in Formel I, entweder mit einem 3-Nitropyrazin mit einer austretenden Gruppe in der 2-Stellung oder mit einem 3-Nitropyridin mit einer austretenden Gruppe in der 4-Stellung in Gegenwart eines organischen Lösungsmittels und einer organischen oder anorganischen Base oder in Gegenwart eines basischen organischen Lösungsmittels umgesetzt wird, wobei die Verbindungen der Formel I in freier Form oder in Form ihrer Salze isoliert werden.

7. Verfahren nach Anspruch 6, dadurch gekennzeichnet, daß die austretende Gruppe des 3-Nitropyrazin-Ausgangsmaterials und entsprechend des 3-Nitropyridin-Ausgangsmaterials ein Chloratom ist und daß die Reaktion in Gegenwart eines niederen Alkohols, vorzugsweise Isopropylalkohol, oder eines polaren organischen Lösungsmittels durchgeführt wird.

8. Pharmazeutische Zusammensetzung zur Verstärkung des therapeutischen Effekts einer Strahlungsbehandlung bösartiger Erkrankungen, dadurch gekennzeichnet, daß die pharmazeutische Zusammensetzung als pharmazeutisch wirksamen Bestandteil eine Verbindung der Formel I nach Anspruch 1 oder ein pharmazeutisch annehmbares Salz davon enthält.

9. Pharmazeutische Zusammensetzung nach Anspruch 8, dadurch gekennzeichnet, daß sie als pharmazeutisch wirksamen Bestandteil eine Verbindung nach einem der Ansprüche 2, 3, 4 oder 5 enthält.

10. Pharmazeutische Zusammensetzung nach Anspruch 8 oder 9, dadurch gekennzeichnet, daß sie weiterhin einen pharmazeutisch annehmbaren Träger enthält.

## Revendications

1. Composé hétérocyclique nitro substitué de formule I

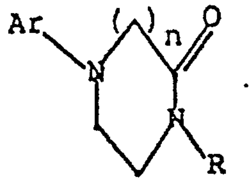

I

dans laquelle

n est le nombre entier 1 ou 2,

R est de l'hydrogène ou un groupe hydroxyéthyl et

Ar est un groupe hétéroaryle choisi parmi les 3-nitro-2-pyrazinyle et 3-nitro-4-pyridyle et les sels des composés de formule I.

2. Un composé selon la revendication 1, qui est la 1-(3-nitro-2-pyrazinyl)pypérazine-3-one.

3. Un composé selon la revendication 1, qui est la 1-(3-nitro-2-pyrazinyl)hexahydro-1H-1,4-diazépine-5-one.

4. Un composé selon la revendication 1, qui est la 1-(3-nitro-2-pyrazinyl)-4-(2-hydroxyéhyl)pipérazine-3-one.

5. Un composé selon la revendication 1, qui est la 1-(3-nitro-4-pyridyl)pipérazine-3-one.

6. Un procédé pour la préparation de composés de formule I

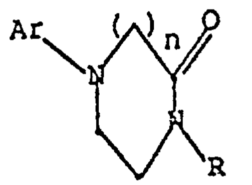

I

selon la revendication 1, dans laquelle

n, R et Ar ont la même signification que spécifiée dans la revendication 1 ou des sels des composés de formule I, caractérisé en ce que le lactame de formule II

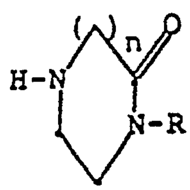

II

dans laquelle

n et R ont la même signification que dans la formule I, est mis à réagir soit avec une 3-nitropyrazine présentant un groupe partant en position 2 soit avec une 3-nitro-pyridine présentant un groupe partant en position 4, en présence d'un solvant organique et d'une base organique ou inorganique, ou bien en présence d'un solvant basique organique, et dans lequel les composés de formule I sont isolés sous forme libre ou de leurs sels.

7. Procédé selon la revendication 6, caractérisé en ce que le groupe partant du matériau de départ, 3-nitro-pyrazine, et du matériau de départ 3-nitropyridine, respectivement est un atome de chlore et que la réaction est mise en œuvre en présence d'un alcool inférieur, de préférence de l'alcool isopropylique ou un solvant polaire organique.

8. Composition pharmaceutique pour améliorer l'effet thérapeutique d'un traitement par radiations des maladies malignes, caractérisée en ce que la composition pharmaceutique renferme comme ingrédient pharmaceutiquement actif, un composé de formule I selon la revendication 1 ou un de ses sels pharmaceutiquement compatible.

9. Composition pharmaceutique selon la revendication 8, caractérisée en ce qu'elle renferme comme ingrédient pharmaceutiquement actif, un composé selon l'une des revendications 2, 3, 4 ou 5.

10. Composition pharmaceutique selon la revendication 8 ou 9, caractérisée en ce qu'elle renferme en outre un support pharmaceutiquement compatible.